# EUROPEAN PATENT APPLICATION

(11) **EP 3 292 824 A1**
(43) Date of publication of application: **14.03.2018**
(21) Application number: 16783153.6
(22) Date of filing: 19.04.2016
(51) Int. Cl.: A61B 17/00, A61B 17/3205

(54) **TISSUE PRESSING TOOL**

(30) Priority: 20.04.2015 JP 2015086247
(71) Applicant: Olympus Corporation, Hachioji-shi, Tokyo 192-8507 (JP)
(72) Inventor: MOTAI Kosuke, Hachioji-shi Tokyo 192-8507 (JP); HATANAKA Takayuki, Hachioji-shi Tokyo 192-8507 (JP)
(74) Representative: Schicker, Silvia
(86) International application number: PCT/JP2016/062388
(87) International publication number: WO 2016/171132

(57) **Abstract**

A tissue pressing tool (310) which can favorably press tissue regardless of the position of a lesion is provided which comprises a shaft (311) which extends in one direction, a tissue pressing part (312) which is pressed against tissue, and a passively flexing part (313) which is connected to the shaft so as to enable flexure of the tissue pressing part in all directions about the axis of the shaft.

## Description

### Technical Field

The present invention relates to a tissue pressing tool used for pressing a tissue in a body cavity.

Priority is claimed on Japanese Patent Application No. 2015-086247, filed April 20, 2015, the content of which is incorporated herein by reference.

### Background Art

As methods of resecting a portion of a hollow organ such as an alimentary canal, a method based on laparotomy in which an abdomen is largely incised, and a method using an endoscope or the like without incising an abdomen are known.

In the method based on the laparotomy, a wide range of resection can be easily performed, while stress given to a patient is large. On the other hand, in the method using the endoscope, the stress given to a patient is small, while the size of a lesion being capable of being resected is limited.

As described above, both of the method based on the laparotomy and the method using the endoscope have disadvantages. Therefore, a tissue resection method in which resection can be performed in a wider range than in the method using the endoscope and the stress given to a patient is smaller than in the method based on the laparotomy is required. In relation to this, non-patent document 1 proposes a technique of using a medical device introduced into a natural opening and a medical device introduced into an abdominal cavity in cooperation.

### Citation List

### Non-Patent Document

[Non-Patent Document 1] Journal of Japan Surgical Society Vol. 115, No. 2, 102-104

### Summary of Invention

### Technical Problem

However, the surgical procedure described in Non-Patent Document 1 is not common because it requires an advanced technology such as hooking up the stomach wall to the abdominal wall and lifting it.

As another method, it has been proposed to press a hollow organ with a forceps introduced into the abdominal cavity and to cut out the target tissue with a medical device introduced into the hollow organ. However, as will be described in detail later, it is sometimes difficult to perform the procedure because there is an area in some hollow organs where it is difficult to be pressed by the forceps introduced into the abdominal cavity.

In view of the above circumstances, an object of the invention is to provide a tissue pressing tool that can suitably press a tissue regardless of the lesion position.

### Solution to Problem

According to a first aspect of the invention, a tissue pressing tool includes: a shaft that extends in one direction; a tissue pressing part that is pressed against a tissue; and a passive bending part that connects the tissue pressing part with the shaft so that the tissue pressing part is capable of bending in all direction around an axis line of the shaft.

According to a second aspect of the invention, in the tissue pressing tool according to the first aspect, the passive bending part may be composed of a ball joint.

According to a third aspect of the invention, in the tissue pressing tool according to the first aspect, the passive bending part may be composed of an elastic body.

According to a fourth aspect of the invention, in the tissue pressing tool according to any one of the first to third aspects, the tissue pressing part may be configured to change an orientation relative to the shaft at a more distal side than the passive bending part.

According to a fifth aspect of the invention, in the tissue pressing tool according to the fourth aspect, the tissue pressing part may have a ratchet at a more distal side than the passive bending part.

According to a sixth aspect of the invention, the tissue pressing tool according to the fourth or fifth may further include: a stopper that limits a variable range of the orientation of the tissue pressing part to the shaft.

According to a seventh aspect of the invention, in the tissue pressing tool according to any one of the fourth to sixth aspects, the tissue pressing part may be configured to actively change the orientation relative to the shaft.

According to an eighth aspect of the invention, in the tissue pressing tool according to any one of the first to seventh aspect, the tissue pressing part may include a bend part that has a curved surface at a distal end part.

According to a ninth aspect of the invention, in the tissue pressing tool according to any one of the first to eighth aspect, at least a part of the tissue pressing part contacting to the tissue may be formed of a biodegradable material.

According to a tenth aspect of the invention, in the tissue pressing tool according to the ninth aspect, the tissue pressing part may include two arms that are connected to the passive bending part and a linear member that is composed of the biodegradable material and is stretched between the two arms.

According to an eleventh aspect of the invention, in the tissue pressing tool according to the ninth or tenth aspect, a part composed of the biodegradable material may have such elasticity as to be flexible.

### Advantageous Effects of Invention

According to the tissue pressing tool of the invention, it is possible to suitably press a tissue regardless of the lesion position.

### Brief Description of Drawings

FIG. 1 is a view showing an example of a first step in a tissue resection method according to a reference example of the present invention.
FIG. 2 is a view showing a tissue pressing tool used for the tissue resection method according to the reference example of the present invention.
FIG. 3 is a view showing an example of a second step in the tissue resection method according to the reference example of the present invention.
FIG. 4 is a view showing an inverted resection target tissue in the second step in the tissue resection method according to the reference example of the present invention.
FIG. 5 is a view showing an example of a third step in the tissue resection method according to the reference example of the present invention.
FIG. 6 is a view showing a tissue pressing tool according to a modification example of the reference example of the present invention.
FIG. 7 is a view showing an example of the second step using the tissue pressing tool according to the modification example of the reference example of the present invention.
FIG. 8 is a schematic view showing a human large bowel.
FIG. 9 is a view showing a tissue pressing tool according to the first embodiment of the present invention.
FIG. 10 is a view showing one process when using the tissue pressing tool according to the first embodiment of the present invention.
FIG. 11 is a view showing one process when using the tissue pressing tool according to the first embodiment of the present invention.
FIG. 12 is a view showing an example of a positional relationship between the tissue pressing tool according to the first embodiment of the present invention and a resection and anastomosis device.
FIG. 13 is a view showing an operation where the positional relationship between the tissue pressing tool and the resection and anastomosis device in FIG. 12 is corrected.
FIG. 14 is a view showing a tissue pressing tool according to the second embodiment of the present invention.
FIG. 15 is a partially sectional view showing a tissue pressing tool according to a modification of the second embodiment of the present invention.
FIG. 16 is a view showing a tissue pressing tool according to the third embodiment of the present invention.
FIG. 17 is a partially sectional view showing a tissue pressing tool according to a modification of the third embodiment of the present invention.
FIG. 18 is a view showing a tissue pressing tool according to a modification of the third embodiment of the present invention.
FIG. 19 is a partially sectional view showing a tissue pressing tool according to a modification of the third embodiment of the present invention.
FIG. 20 is a view showing one process when using the tissue pressing tool in FIG. 19.
FIG. 21 is a partially enlarged view showing a resection and anastomosis device corresponding to the tissue pressing tool according to a modification of the present invention.

### Description of Embodiments

### (Reference example: tissue resection method)

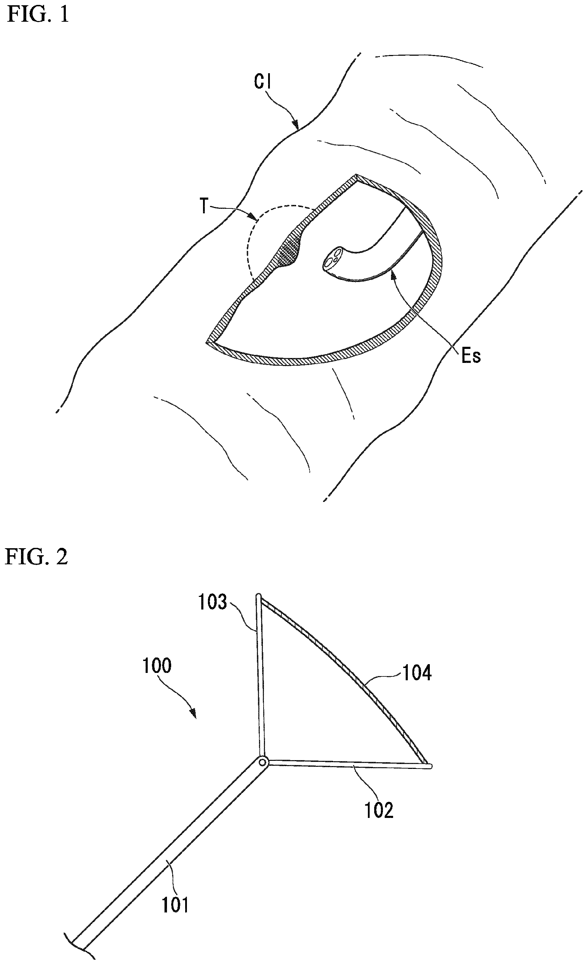

First, the outline of a tissue resection method performed using a tissue pressing tool of the invention will be described with reference to FIGS. 1 to 7. Hereinafter, a tissue resection method related to a reference example of the invention will be described, taking as an example, a case where tissue in a certain region including a lesioned site of the large intestine, which is a hollow organ, as a target is resected over all layers.

In the following description, an operator who approaches resection target tissue from a lumen side of the large intestine is referred to as a first operator, and an operator who approaches the resection target tissue from an abdominal cavity (body cavity) side is referred to as a second operator.

FIG. 1 is a view illustrating an example of a first step in the tissue resection method related to the reference example. First, as illustrated in FIG. 1, the first operator introduces observation means, such as an endoscope Es, into a large intestine Cl, and observes the inside of the large intestine Cl with the observation means to identify the position and the range of resection target tissue T (first step).

After the position and the range of the resection target tissue T are identified, the first operator shows the position and the range of the resection target tissue T to the second operator in such a manner that the position and the range can be confirmed from the abdominal cavity side. A specific method for showing the position and the range is not particularly limited, and a well-known method can be appropriately selected and can be used. For example, the above method includes protruding to the abdominal cavity side by pressing a portion of the resection target tissue T with an endoscope, a treatment tool inserted into the endoscope, or the like, performing inking on a portion of resection target tissue T, illuminating a portion of the resection target tissue T in a visually recognizable manner from the abdominal cavity side, and the like.

The second operator that has confirmed the position of the resection target tissue T inserts a tissue pressing tool into an access port formed in an abdominal wall, and introduces the tissue pressing tool into the abdominal cavity. The method of forming the access port is not particularly limited, and can be performed by, for example, indwelling a trocar in the abdominal wall.

FIG. 2 is a view illustrating a tissue pressing tool 100 used for the tissue resection method related to the reference example. The tissue pressing tool 100 has a structure in which a pair of arms 102 and 103 is provided at the distal end of a rod-shaped main body 101. A linear member (tissue contacting part) 104 is stretched between distal end parts of the pair of arms 102 and 103. The linear member 104 is a biodegradable member that is formed of a biodegradable material which is decomposed and absorbed without causing inflammation or the like in a living body and that has bendable flexibility.

Proximal end parts of the pair of arms 102 and 103 are turnably connected to a distal end part of the main body 101, and are capable of maintaining an angle formed between the arms 102 and 103 and the main body 101 at a constant holding force. For this reason, it is possible to make the pair of arms 102 and 103 parallel to the main body 101 and make the entire tissue pressing tool 100 linear. Additionally, it is possible to open the pair of arms 102 and 103 and stretch the linear member 104 linearly. The pair of arms 102 and 103 may be configured to be openable and closable with the second operator's hands.

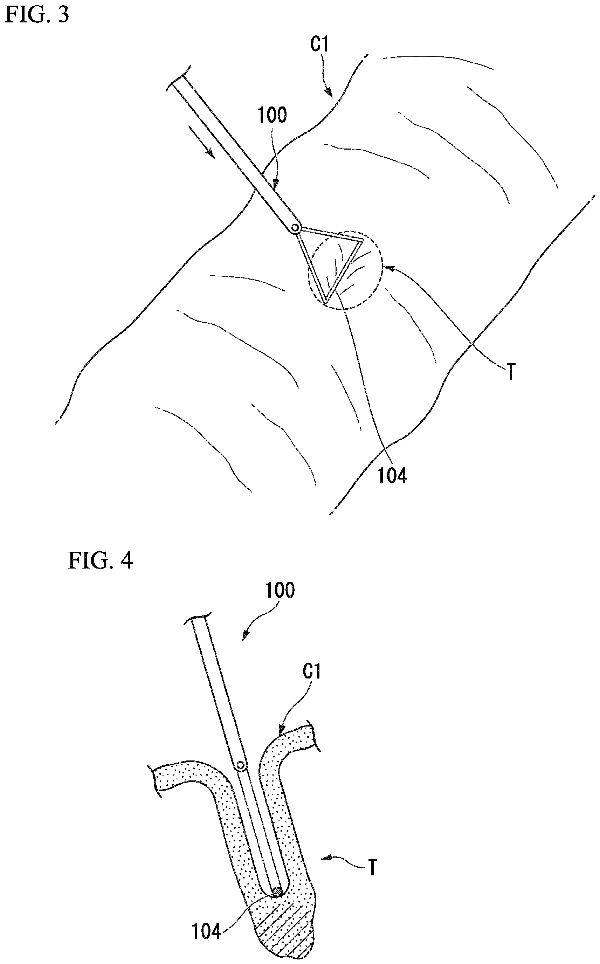

The second operator presses the arms 102 and 103 against the abdominal wall within the abdominal cavity, or operates arms 102 and 103 with his/her hands, thereby opening the arms 102 and 103 of the tissue pressing tool 100. FIG. 3 is a view illustrating an example of a second step in the tissue resection method related to the reference example. FIG. 4 is a view illustrating an inverted resection target tissue in the second step.

As illustrated in FIG. 3, the second operator brings the linear member 104 into contact with the resection target tissue T shown by the first operator, and presses the resection target tissue with the tissue pressing tool 100. Due to this operation, as illustrated in FIG. 4, the resection target tissue T is deformed so as to protrude to the inside of the large intestine Cl, and is folded to a lumen side of the large intestine Cl, with a site where the linear member 104 is in contact as a bend line (second step). In the following description, a state where the tissue is folded in this way is referred to as an "inverted state" or an "inversion state". The resection target tissue T brought into the inversion state sandwiches only the linear member 104 therein on the abdominal cavity side, and the arms 102 and 103 are not sandwiched in the resection target tissue T.

The first operator resects the inverted resection target tissue T over all layers from the lumen side of the large intestine Cl. When the resection target tissue T is resected over all layers, a hole communicating with the abdominal cavity is formed in the hollow organ. Therefore, this hole is closed by being anastomosed or sutured (hereinafter referred to "anastomosis or the like"). A process in which the resection of this tissue, the anastomosis or the like of the hole is performed is a third step.

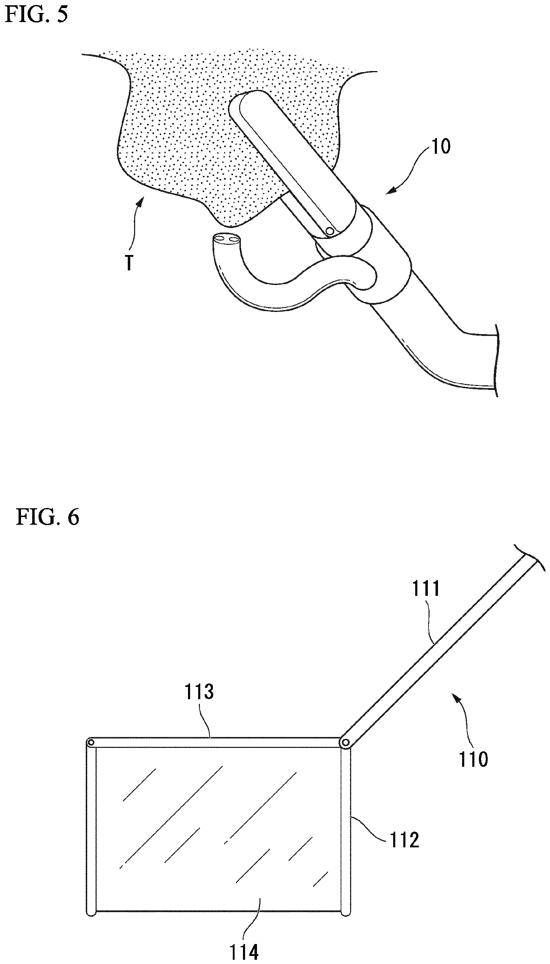

Although the resection of the tissue, the anastomosis of the hole or the like may be separately performed using different medical instruments, it is possible to simultaneously perform the resection of the tissue, the anastomosis or the like of the hole when a well-known linear stapler or circular stapler (hereinafter generically referred to as a "stapler or the like"), a high-frequency anastomosis machine, or the like is used. FIG. 5 is a view illustrating an example of the third step in the tissue resection method related to the reference example. In the example of FIG. 5, the resection of the tissue, the anastomosis or the like of the hole is performed using a linear stapler 10.

Thus, the tissue resection method related to the reference example is completed.

In a case where the stapler or the like is used in the third step, a portion of the linear member 104 may be locked to staples and may remain on an outer surface of the hollow organ. However, since the linear member 104 is formed of the biodegradable material, the linear member disappears without causing inflammation or the like with the lapse of time.

As described above, according to the tissue resection method of the reference example, the resection target tissue is inverted to the lumen side of the hollow organ by the tissue pressing tool introduced into the abdominal cavity in the second step. Therefore, it is easy to resect the resection target tissue in spite of the approach from the lumen side.

That is, in a case where the resection target tissue is not inverted, it is necessary to perform the resection at a cutting line of such a shape that the resection target tissue is surrounded, and it is complicated and difficult to perform this from the lumen side. On the other hand, in a case where the resection target tissue is inverted, the shape of the cutting line just has to be set such that the resection target tissue is surrounded when the inverted resection target tissue is deployed. Therefore, the resection may be performed at one or two linear cutting lines or one circular-arc cutting line and can be easily performed using the stapler or the like from the lumen side.

In addition, in the tissue resection method of the reference example, the biodegradable member that comes in contact with the resection target tissue is not limited to the above linear member.

FIG. 6 is a view illustrating an example of a tissue pressing tool related to a modification example of the reference example. As illustrated in FIG. 6, in a tissue pressing tool 110 of the modification example, a sheet-like biodegradable member (tissue contacting part) 114 is attached between a first arm 112 and a second arm 113. Since the first arm 112 and the second arm 113 are parallel to a main body 111 and are linearly deformable over the tissue pressing tool 110 and the biodegradable member 114 is bendable, these arms can be easily introduced into the abdominal cavity from the access port. As the sheet-like biodegradable member, for example, NEOVEIL (trade name) manufactured by Gunze, Ltd. using polyglycolic acid as a material or the like can be used.

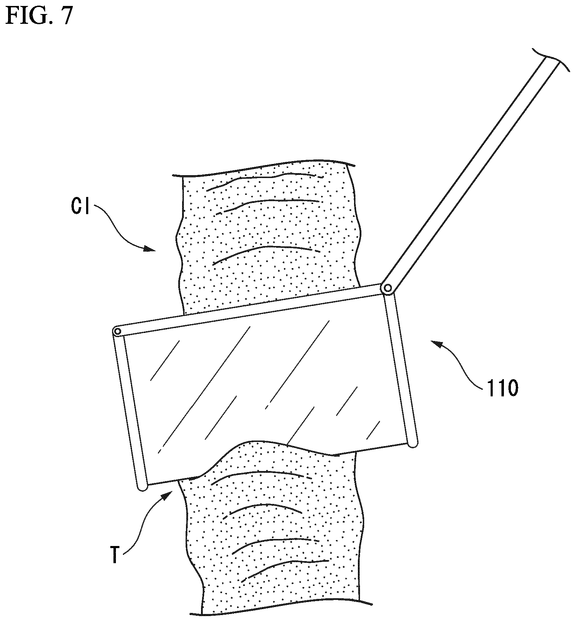

FIG. 7 is a view illustrating an example of the second step using the tissue pressing tool related to the modification example of the reference example. As illustrated in FIG. 7, the second step is performed on the large intestine Cl using the tissue pressing tool 110.

Moreover, the tissue pressing tool is not limited to one to which the biodegradable member is attached as described above. For example, both ends of the linear member 104 may be grasped by two well-known grasping forceps, and the linear member stretched linearly may be pressed against the resection target tissue. Additionally, the biodegradable member 114 may be grasped by one grasping forceps to press the biodegradable member 114 against the resection target tissue. In these cases, the grasping forceps constitutes a portion of the tissue pressing tool.

Additionally, a medical instrument that performs the third step is not limited to one using a staple. For example, the resection of the tissue and the anastomosis of the hole may be performed by the application of energy.

### (First embodiment: tissue pressing tool)

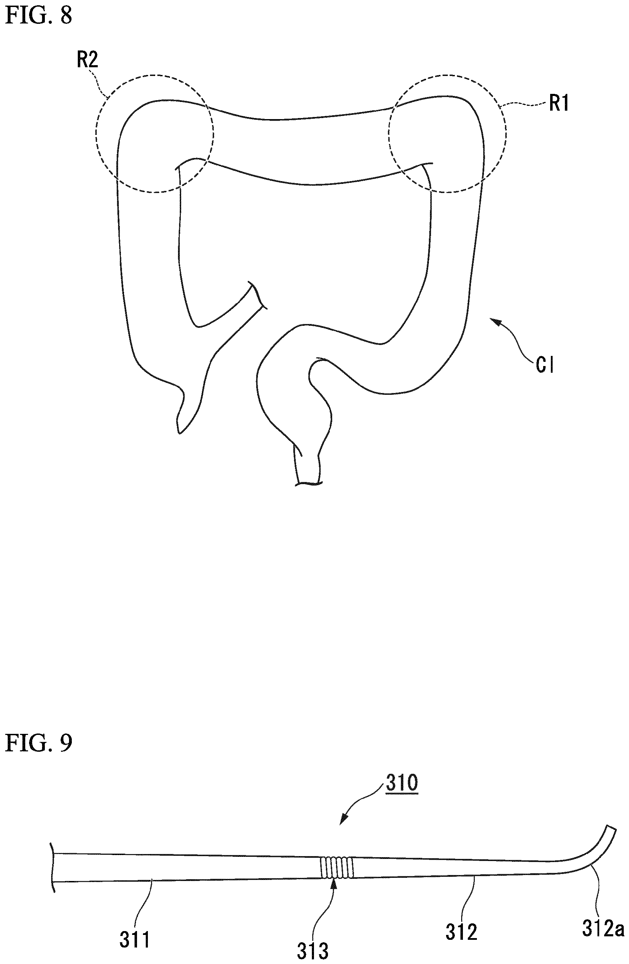

Next, a first embodiment of the invention will be described with reference to FIGS. 8 to 13. In the present embodiment, a tissue pressing tool used for suitably performing the tissue resection method described in the reference example will be described.

In the subsequent description, the same components as those already described will be designated by the same reference signs and a redundant description thereof will be omitted.

FIG. 8 is a diagram schematically showing a human large bowel. As described above, the tissue resection method of the present invention is targeted for the luminal organ such as the stomach and the intestine, but when the large bowel Cl is targeted, it may be difficult to execute the second step and the third step in the splenic flexure indicated by the area R1 and its surroundings and the hepatic flexure indicated by the area R2 and its surroundings in FIG. 8.

That is, when the tissue pressing tool is introduced from a general access port position in a laparoscopic surgical operation and a procedure is performed in combination with a circular stapler type resection and anastomosis device, there is a case in which it is difficult to coincide the longitudinal direction of the gap between the resection and anastomosis device main body and the anvil part in the resection and anastomosis device (substantially equal to the radial direction of the treatment part) and the longitudinal direction of the contacting portion between the tissue pressing tool and the resection-target tissue in the above-mentioned position (area R1 and R2). When the above two directions do not coincide, it is impossible to bring the resection and anastomosis main body and the anvil part sufficiently close while keeping a state in which the tissue is pressed by bringing the tissue pressing tool and the resection and anastomosis device into contact with each other, and it becomes difficult to make a resection of tissue and an anastomosis of hole.

On the other hand, it is conceivable to move the position of the resection-target tissue in the abdominal cavity, but it is difficult to move it since the above-mentioned position (area R1 and R2) is fixed with a ligament. Also, if peeling, cutting of ligaments, blood vessels or the like is performed in order to move it, the stress given to a patient becomes large.

In addition, it is conceivable to form an access port at a position suitable for the tissue resection method, but there is a possibility that, in the case of shifting to a usual laparoscopic procedure due to an unexpected situation or the like, the laparoscopic procedure may become difficult by such access port. Furthermore, forming an access port dedicated to the tissue pressing tool in addition to the general access port increases the stress given to a patient.

The tissue pressing tool of the present embodiment makes it possible to perform the tissue resection method of the present invention suitably even in regions such as the splenic flexure (area R1), the hepatic flexure (area R2), or the like, and it can be suitably used in regions other than the splenic flexure, the hepatic flexure, or the like. Each embodiment of the tissue pressing tool of the present embodiment will be described below.

FIG. 9 is a view showing the tissue pressing tool 310 according to this embodiment. The tissue pressing tool 310 includes a rigid shaft 311 extending in one direction, a pressing member (tissue pressing part) 312 connected to the shaft 311, a coil spring (elastic body, passive bending part) 313 that connects the shaft 311 to the pressing member 312. The pressing member 312 can be bent in all directions around the axis of the shaft 311 with respect to the shaft 311 by being connected with the shaft 311 via the coil spring 313.

The pressing member 312 is formed of a biodegradable material and is a rod-like or belt-like member having a rigidity enough to hold the shape even when pressed against the resection target tissue. The pressing member 312 has a bend part 312a which is slightly bent and has a curved surface at the distal end part.

It suffices that the coil spring 313 has elasticity enough to generate passive bending which will be described later, and the material, shape, and the like are not particularly limited.

The operation when the tissue pressing tool 310 configured as described above is used will be described using an example when used with a circular stapler type resection and anastomosis device. The tissue pressing tool 310 is substantially linear in a state in which no force is applied, so it can be easily inserted into the access port and introduced into the abdominal cavity.

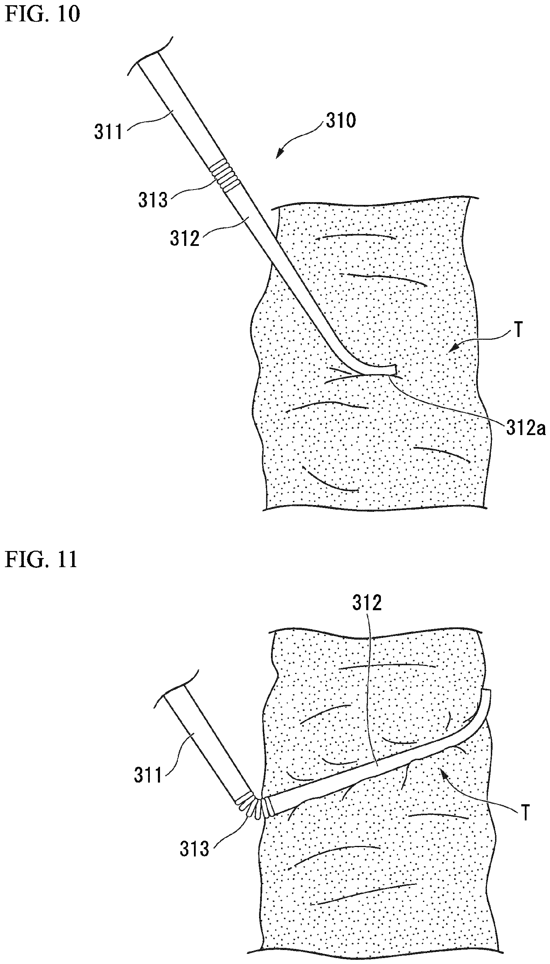

FIG. 10 and FIG. 11 are views showing a process when using the tissue pressing tool according to the first embodiment. When performing the second step using the tissue pressing tool 310, the second operator brings the curved surface outside the bending shape at the bend part 312a into contact with the resection target tissue T as shown in FIG. 10. When the second operator makes the tissue pressing tool 310 advance, the resection target tissue T is pressed by the bend part 312a. Further, a reaction force received from the resection target tissue T acts on the bend part 312a to cause elastic deformation of the coil spring 313. As a result, as shown in FIG. 11, the pressing member 312 pivots so as to form an angle with the shaft 311, and the pressing member 312 comes into contact with the resection target tissue T in a longer range. By further pressing the resection target tissue T in this state, the resection target tissue T can be linearly pressed to be in an inverted state.

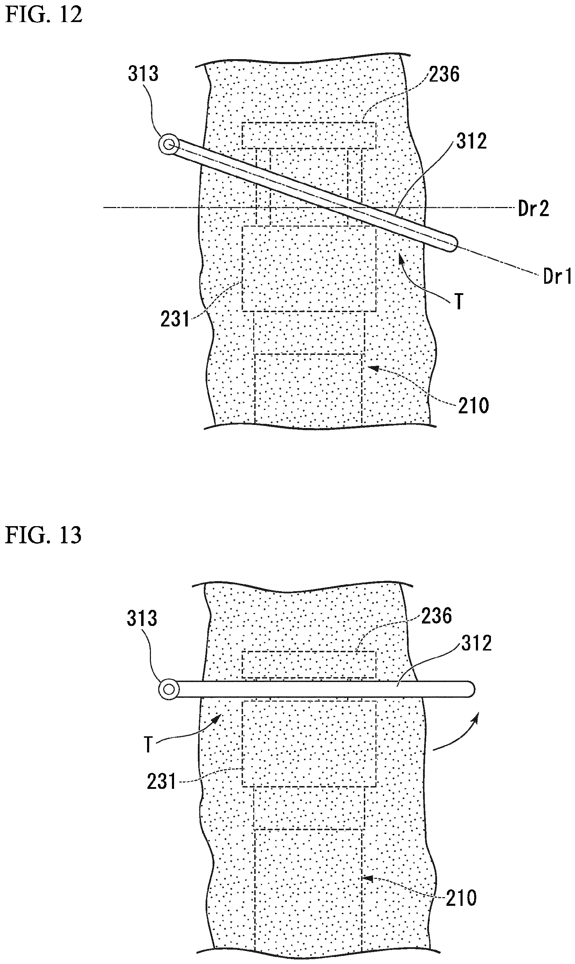

FIG. 12 is a view showing an example of the positional relationship between the tissue pressing tool and the resection and anastomosis device according to the first embodiment, and FIG. 13 is a view showing an operation in which the positional relationship between the tissue pressing tool and the resection and anastomosis device in FIG. 12 is corrected. At the end of the second step, as shown in FIG. 12, a direction Dr1 in which the contacting portion between the pressing member 312 and the resection target tissue T extends may not be parallel to a direction Dr2 of the width of the treatment part that includes the resection and anastomosis device body 231 and the anvil part 236 in the resection and anastomosis device 210. In this case, when the first operator brings the anvil part 236 closer to the main body 231 while the second operator holds the pressing state, the pressing member 312 located between the anvil part 236 and the main body 231 is pressed in accordance with close of the anvil part 236 to the main body 231. As a result, the coil spring 313 is elastically deformed, and as shown in FIG. 13, the pressing member 312 passively bends (rotates, moves) with the coil spring 313 as a fulcrum. Due to the passive bending (rotation, movement) of the pressing member 312, the direction Dr1 and the direction Dr2 finally become parallel and the resection target tissue T is suitably sandwiched between the anvil part 236 and the main body 231. In this state, removal of the tissue and anastomosis of hole in the third step are performed.

As described above, according to the tissue pressing tool 310 of the present embodiment, a passive bending part including the coil spring 313 is provided between the shaft 311 and the pressing member 312. Thereby, when the resection target tissue can be made inverted between the anvil part of the resection and anastomosis device and the resection and anastomosis device main body, even if the direction Dr1 and the direction Dr2 are not parallel, a state in which the direction Dr1 and the direction Dr2 are parallel can be realized semi-automatically by bringing the anvil part and the resection and anastomosis device main body close to each other. That is, since the pressing member 312 can be bent (rotated, moved) in all directions about the axis of the shaft 311 with respect to the shaft 311 by deformation of the coil spring 313, the direction Dr1 and the direction Dr2 are corrected so as to be parallel whatever relationship between the direction Dr1 and the direction Dr2 is. Therefore, even if the resection target tissue is located at a site such as the splenic flexure (area R1) or the hepatic flexure (area R2), the second step and the third step can be easily performed using the access port formed at a general position, and it is not necessary for the second operator to adjust the angle of the pressing member 312, cooperate operation between the first operator and the second operator, and the like.

### (Second embodiment)

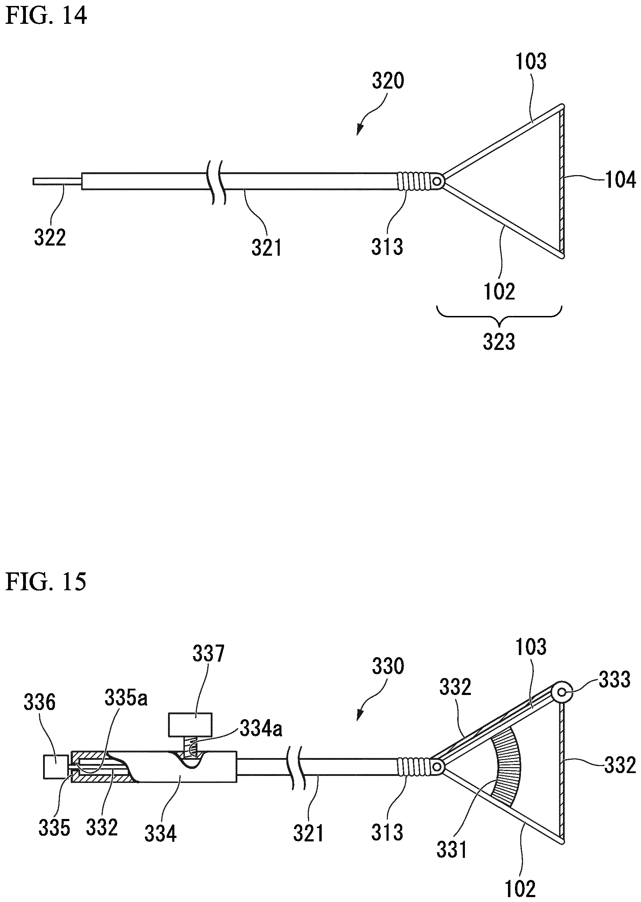

Next, a second embodiment of the present invention will be described with reference to FIGS. 14 and 15. FIG. 14 is a view showing the tissue pressing tool 320 according to the second embodiment. The tissue pressing tool 320 includes arms 102, 103 and a linear member 104 similarly to the tissue pressing tool 100 described in the reference example. The tissue pressing part 323, which is composed of the arms 102, 103 and the linear member 104, and the shaft 321 are connected via a coil spring 313.

The shaft 321 is hollow, and a wire 322 for opening and closing the arms 102, 103 is inserted therein. The distal end portion of the wire 322 and the proximal end portion of the arms 102, 103 are connected by a known link mechanism or the like, and the arms 102, 103 can be opened and closed by advancing and retracting the wire 322 with respect to the shaft.

The operation at the time of use of the tissue pressing tool 320 is basically the same as that of the tissue pressing tool 100 of the reference example, and the operation of the coil spring 313 is the same as that of the tissue pressing tool 310 of the first embodiment. The tissue pressing tool 320 according to the present embodiment having such a configuration exhibits the same advantage as the tissue pressing tool 310 according to the first embodiment.

FIG. 15 is a partially broken view showing a tissue pressing tool according to a modified example of the present embodiment. The tissue pressing tool 330 according to the modified example of the present embodiment does not have the wire 322. A deployment spring 331 is attached between the arms 102, 103, and urges the pair of arms 102, 103 to open. One end of the linear member 332 is fixed to the distal end of the arm 102. The linear member 332 is hung on a pulley 333 attached to the tip of the arm 103, and then passes through the coil spring 313 and the shaft 321 to protrude from the proximal end of the shaft 321.

The proximal end side of the shaft 321 is inserted into the adjustment sleeve 334. The adjustment sleeve 334 has a bottom surface 335 on the proximal end side. The linear member 332 is drawn out from a hole 335a formed in the bottom surface 335. A stopper 336 is fixed to the end of the linear member 332 drawn out from the hole 335a to prevent the linear member 332 from coming out of the hole 335a into the adjustment sleeve 334. A through hole 334a communicating with the inner cavity is provided on the outer peripheral surface of the adjustment sleeve 334, and a screw 337 is attached to the through hole 334a. When the screw 337 is loose, the adjustment sleeve 334 can advance and retreat relative to the shaft 321. When the screw 337 is tightened, the positional relationship between the adjustment sleeve 334 and the shaft 321 can be fixed.

The operation at the time of using the tissue pressing tool 330 will be described. When the linear member 332 is drawn to the proximal end side of the adjustment sleeve 334, the arms 102, 103 are closed. As a result of this operation, the tissue pressing tool 330 becomes linear as a whole and can be inserted into and removed from the access port.

When the pulling of the linear member 332 is loosened, the arms 102, 103 are opened by the deployment spring 331 as shown in FIG. 15. The opening width of the arms 102, 103 can be adjusted by changing the position of the linear member 332 with respect to the shaft 321, and it is possible to maintain the adjusted state by tightening the screw 337.

The tissue pressing tool 330 having such a structure exhibits the same advantage as the tissue pressing tool 310 of the first embodiment.

In the tissue pressing tool 330, it is possible to change the length of the linear member 322 between the arms 102, 103 according to the resection target tissue. Tension is constantly applied to the linear member 322 between the arms by the deployment spring 331 regardless of the length of the linear member 322 between the arms. Further, since the end portion of the linear member 332 is drawn to the proximal end side of the shaft 321, even if the length of the linear member 322 is adjusted, the linear member 322 does not slacken on the distal end side of the tissue pressing tool 330. As a result, it is possible to limit situations in which the linear member entangles with other instruments or the like in the body.

Furthermore, since the adjustment sleeve 334 and the screw 337 are provided, it is possible to easily maintain the closed state, the opening width, and the like of the arms 102, 103, and the operability is good.

### (Third embodiment)

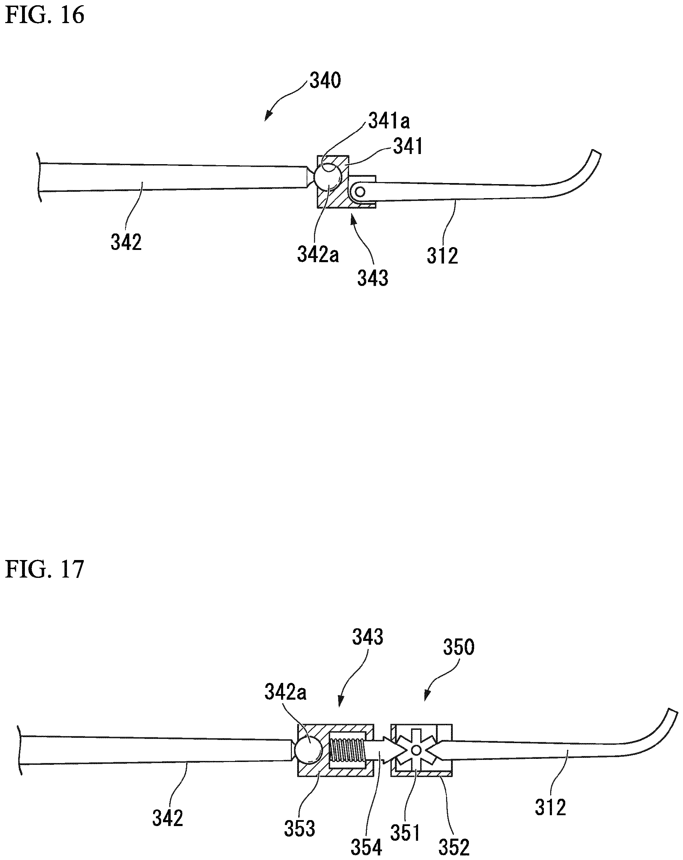

Next, a third embodiment of the present invention will be described with reference to FIGS. 16 to 20. FIG. 16 is a partial cross-sectional view of the tissue pressing tool 340 according to the third embodiment. In the tissue pressing tool 340, a passive bending part is configured by a ball joint 343 instead of a coil spring.

The proximal end portion of the pressing member 312 is rotatably supported by the block 341. The distal end portion 342a of the shaft 342 is formed in a spherical shape and is fitted into a recessed portion 341a formed in the block 341. That is, the ball joint 343 is configured by the distal end portion 342a and the block 341. The shaft 342 and the pressing member 312 are connected via the ball joint 343.

The operation when the tissue pressing tool 340 is used will be described. When the resection target tissue is pressed by the pressing member 312, the pressing member 312 passively rotates with respect to the block 341 by the reaction force received from the resection target tissue, and changes the angle formed with the shaft 342 (direction with respect to the shaft 342). When the pressing member 312 pivots about 90 degrees with respect to the block 341, the pressing member 312 contacts the block 341 and stops rotating further. That is, the block 341 functions as a stopper defining a variable range of the orientation of the pressing member 312 with respect to the shaft 342. The operation of the passive bending part comprising the ball joint 343 is substantially the same as in the first embodiment.

The tissue pressing tool 340 of this embodiment having such a configuration has the same advantage as the tissue pressing tool 310 of the first embodiment.

Further, since the pressing member 312 is pivotally attached to the block 341 on the distal end side of the ball joint 343, fine adjustment of the direction of the pressing member 312 with respect to the shaft 342 can be performed when the second step is executed. Further, since the block 341 functions as a stopper, a situation is hard to happen in which the pressing member 312 rotates too much and it becomes difficult to operate it. In the tissue pressing tool 340, the angle of the pressing member 312 at which the block 341 starts to function as a stopper can be adjusted by appropriately setting the shape of the block 341.

FIG. 17 is a partial cross-sectional view of a tissue pressing tool according to a modified example of the present embodiment.

In the tissue pressing tool 350 according to the modified example, a ratchet 351 is provided at the proximal end portion of the pressing member 312. The pressing member 312 is pivotally attached to the first member 352 by pivotally supporting the ratchet 351.

The distal end portion 342a of the shaft 342 is formed in a spherical shape and is fitted into the second member 353. The ball joint 343 is configured by the distal end portion 342a and the second member 353. A ratchet pin 354 urged to be pressed against the teeth of the ratchet 351 is attached to the second member 353. The first member 352 and the second member 353 are integrally connected via a ratchet pin 354 by a link (not shown).

When the resection target tissue is pressed by the pressing member 312 in the tissue pressing tool 350, the pressing member 312 changes its orientation with respect to the shaft 342 as the ratchet 351 rotates with respect to the ratchet pin 354. That is, the angle formed by the shaft 342 and the pressing member 312 changes by one tooth of the ratchet 351, and the angle is maintained even when the pressing member 312 is separated from the resection target tissue.

The operation of the passive bending part comprising the ball joint 343 is substantially the same as in the first embodiment.

The tissue pressing tool 350 having such a configuration exhibits the same advantage as the tissue pressing tool 310 of the first embodiment.

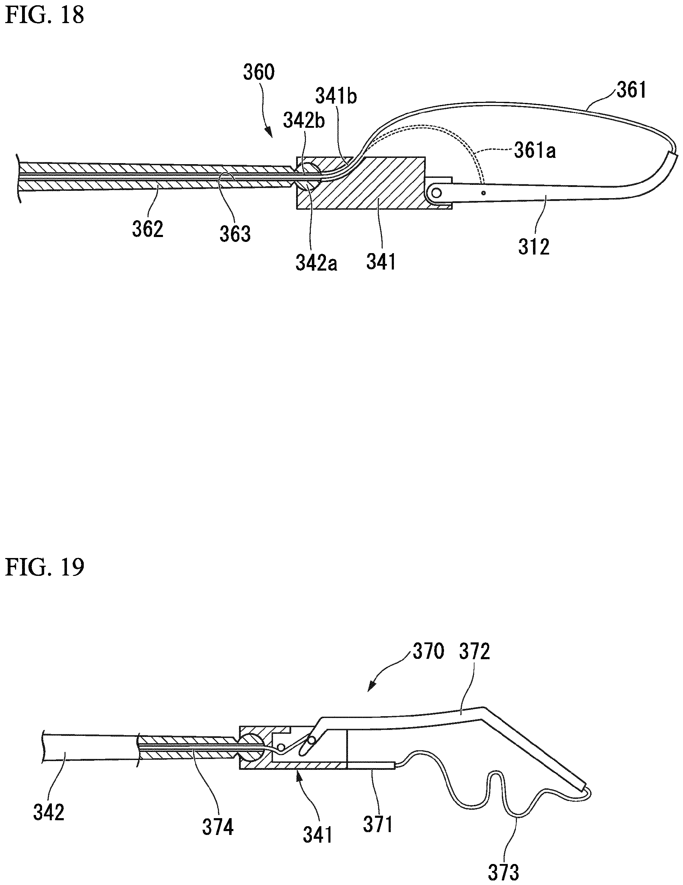

FIG. 18 is a cross-sectional view showing a tissue pressing tool according to another modification of the present embodiment.

In the tissue pressing tool 360 of this modified example, a traction member 361 is connected to the distal end portion of the pressing member 312. The shaft 362 has substantially the same shape as the shaft 342, but a through hole 363 is formed along its axis. The traction member 361 passes through the inside of the block 341 and is passed through the shaft 362 from the distal end side opening of the through hole 363. Other points are the same as the tissue pressing tool 340. It should be noted that the traction member is not necessarily connected to the distal end portion of the pressing member but may be any position as long as the pressing member is caused to rotate. For example, it may be connected to a part of the pressing member like the pressing member 361a (dotted line) in FIG. 18.

The tissue pressing tool 360 having such a structure exhibits the same advantage as the tissue pressing tool 310 of the first embodiment.

In addition, by towing the traction member 361, the pressing member 312 can be pivoted to actively adjust the orientation with respect to the shaft 362, so that the second step can be suitably performed. Further, the pressing member 361 is disposed along the central axis of the shaft 362, and the diameter of the hole 341b through which the pressing member 361 is inserted in the block 341 is sufficiently large with respect to the diameter of the hole 342b through which the traction member 361 is inserted at the distal end portion 342a of the shaft, so even if the traction member 361 is towed, it hardly hinders the operation of the ball joint.

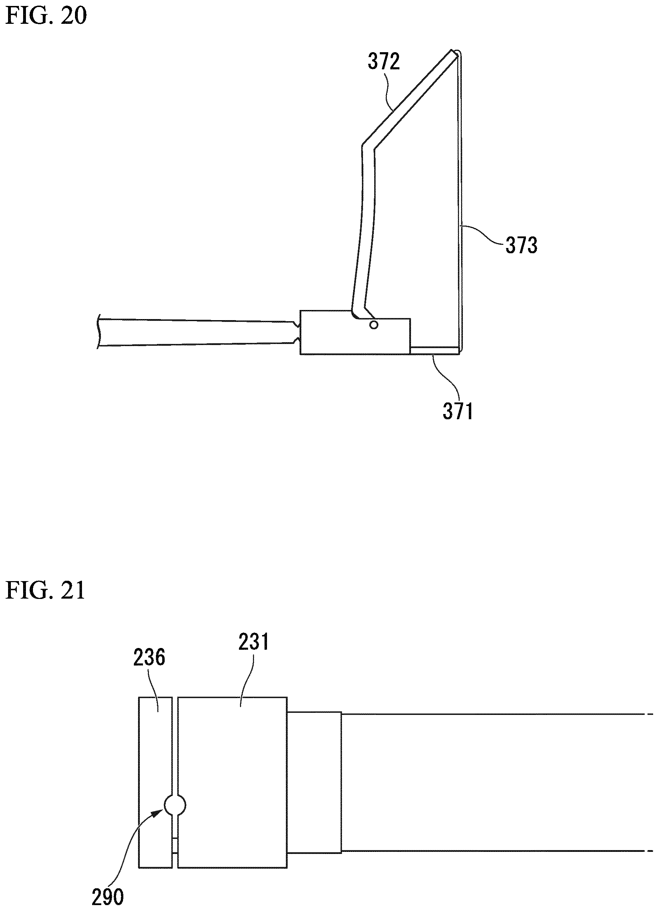

As a tissue pressing tool according to another modification of the present embodiment, a structure that includes an arm that opens and closes may be used. FIG. 19 is a partial cross-sectional view of a tissue pressing tool according to this modification. FIG. 20 is a view showing one process when the tissue pressing tool of FIG. 19 is used. As shown in FIG. 19, the tissue pressing tool 370 of this modification includes a first arm 371 fixed to the block 341 and extending substantially in parallel with the shaft 342, and a second arm 372 pivotally attached to the block 341. A linear member 373 is stretched between the first arm 371 and the second arm 372.

A traction member 374 is connected to the proximal end side of the second arm 372 and is passed through the shaft 342. When the pressing member 374 connected to the second arm 372 is towed from the proximal end side of the shaft 342 in the tissue pressing tool 370, the second arm 372 is rotated, and as shown in FIG. 20, the pair of arms 371 and 372 has a substantially U-shaped configuration, and tension is applied to the linear member 373.

Instead of the first arm 371 and the second arm 372, the tissue pressing tool 370 of the present modification may be provided with the arms 102, 103 of the above-described second embodiment. Conversely, the tissue pressing tool 320 of the second embodiment may be provided with the first arm 371 and the second arm 372 of this modification.

In the tissue pressing tool of the present invention, when the angle between the pressing member and the shaft is changeable at a site different from the passive bending part, if the passive bending part moves before that different site moves, there is a possibility that the operation becomes difficult. Therefore, it is preferable to set the amount of force required for the operation of the passive bending part to be larger than the amount of force required for changing the angle at another portion. Alternatively, a configuration may be employed in which the passive bending part is housed in an outer sheath or the like and held so as not to be bent until the angle adjustment of the pressing member is completed and then the outer sheath or the like is removed to cause passive bending.

While the respective embodiments of the invention have been described above, the technical scope of the invention is not limited to the above embodiments. Combinations of constituent elements can be changed, various alternations can be added to the respective constituent elements, or omissions can be made, without departing from the concept of the invention.

For example, when a member, which has elasticity to the extent that it can be expanded or contracted, such as rubber is used as a linear member formed of a biodegradable material, it is easy to assemble so as to apply tension, making it easier to manufacture. In addition, in the case of a structure that opens and closes an arm with a linear member stretched, it is preferable since the slack amount of the linear member can be reduced.

Additionally, in the tissue pressing tool of the invention, the tissue pressing part may not be formed without using the biodegradable material. In this case, it is necessary to withdraw the tissue pressing part from the resection and anastomosis device before performing the third step. FIG. 21 is a partially enlarged view of the resection and anastomosis device corresponding to the tissue pressing tool related to a modification example of the invention. In the example of FIG. 21, the resection and anastomosis device main body 231 and the anvil part 236 are provided with a relief part 290 according to the dimensions of the tissue pressing parts. In a case where this resection and anastomosis device is used, the third step may be performed after the tissue pressing part is pulled out and withdrawn from the relief part in a state where the resection target tissue inverted by the main body and the anvil part is sandwiched and sufficiently held without causing the tissue pressing part to interfere with the main body and the anvil part.

### Industrial Applicability

The invention can be widely applied to tissue pressing tools used for pressing a tissue in a body cavity, and makes it possible to suitably press a tissue regardless of the lesion position.

### Reference Signs List

10: LINEAR STAPLER
100, 110, 310, 320, 330, 340, 350, 360, 370: TISSUE PRESSING TOOL
101: TISSUE PRESSING TOOL MAIN BODY
102, 103, 112, 113: ARM
104, 332, 373: LINEAR MEMBER (TISSUE CONTACTING PART)
114: BIODEGRADABLE MEMBER (TISSUE CONTACTING PART)
210: RESECTION AND ANASTOMOSIS DEVICE
231: RESECTION AND ANASTOMOSIS DEVICE MAIN BODY
236: ANVIL PART (SECOND MEMBER)
290: RELIEF PART
311,321,342,362: SHAFT
312: PRESSING MEMBER (TISSUE PRESSING PART)
312a: BEND PART
313: COIL SPRING (PASSIVE BENDING PART)
322: WIRE
323: TISSUE PRESSING PART
331: DEPLOYMENT SPRING
333: PULLEY
334: ADJUSTMENT SLEEVE
334a: THROUGH HOLE
335: BOTTOM SURFACE OF ADJUSTMENT SLEEVE
335a: HOLE IN BOTTOM SURFACE OF ADJUSTMENT SLEEVE
336: STOPPER
337: SCREW
341: BLOCK
341a: RECESSED PORTION
341b: HOLE OF BLOCK
342a: DISTAL END PORTION OF SHAFT
342b: HOLE OF SHAFT
343: BALL JOINT (PASSIVE BENDING PART)
351: RATCHET
352: FIRST MEMBER
353: SECOND MEMBER
354: RATCHET PIN
361, 361a, 374: TRACTION MEMBER
363: THROUGH HOLE OF SHAFT
371: FIRST ARM
372: SECOND ARM
C1: LARGE INTESTINE
Dr1, Dr2: DIRECTION
Es: ENDOSCOPE
R1: AREA (SPLENIC FLEXURE)
R2: AREA (HEPATIC FLEXURE)
T: RESECTION TARGET TISSUE

## Claims

1. A tissue pressing tool comprising:
a shaft that extends in one direction;
a tissue pressing part that is pressed against a tissue; and
a passive bending part that connects the tissue pressing part with the shaft so that the tissue pressing part is capable of bending in all direction around an axis line of the shaft.

2. The tissue pressing tool according to Claim 1, wherein the passive bending part is composed of a ball joint.

3. The tissue pressing tool according to Claim 1, wherein the passive bending part is composed of an elastic body.

4. The tissue pressing tool according to any one of Claims 1 to 3, wherein the tissue pressing part is configured to change an orientation relative to the shaft at a more distal side than the passive bending part.

5. The tissue pressing tool according to Claim 4, wherein the tissue pressing part has a ratchet at a more distal side than the passive bending part.

6. The tissue pressing tool according to Claim 4 or 5, further comprising:
a stopper that limits a variable range of the orientation of the tissue pressing part to the shaft.

7. The tissue pressing tool according to any one of Claims 4 to 6, wherein the tissue pressing part is configured to actively change the orientation relative to the shaft.

8. The tissue pressing tool according to any one of Claims 1 to 7, wherein the tissue pressing part includes a bend part that has a curved surface at a distal end part.

9. The tissue pressing tool according to any one of Claims 1 to 8, wherein at least a part of the tissue pressing part contacting to the tissue is formed of a biodegradable material.

10. The tissue pressing tool according to Claim 9, wherein the tissue pressing part includes two arms that are connected to the passive bending part and a linear member that is composed of the biodegradable material and is stretched between the two arms.

11. The tissue pressing tool according to Claim 9 or 10, wherein a part composed of the biodegradable material has such elasticity as to be flexible.
